# EUROPÄISCHE PATENTSCHRIFT

(11) **EP 0 623 587 B1**
(45) Veröffentlichungstag und Bekanntmachung des Hinweises auf die Patenterteilung: **21.02.1996**
(21) Anmeldenummer: 94103840.8
(22) Anmeldetag: 12.03.1994
(51) Int. Cl.: C07C 227/08, C07C 229/16, C07C 233/36, C07C 233/38, C07C 231/12, C11D 1/10

(54) **Verfahren zur Herstellung von amphoteren Tensiden**
Method for the preparation of amphoteric detergents
Procédé pour la préparation des détergents amphotériques

(30) Priorität: 26.03.1993 DE 4309900
(43) Veröffentlichungstag der Anmeldung: 09.11.1994
(73) Patentinhaber: Th. Goldschmidt AG, D-45127 Essen (DE)
(72) Erfinder: Grüning, Burghard, Dr., D-45134 Essen (DE); Weitemeyer, Christian, Dr., D-45134 Essen (DE)

(56) Entgegenhaltungen:
- EP-A- 0 020 907
- EP-A- 0 557 835
- WO-A-91/08193

## Beschreibung

Die Erfindung betrifft ein Verfahren zur Herstellung von amphoteren Tensiden durch Umsetzung von Aminen mit Chloressigsäure oder deren Salzen in wäßriger Lösung bei erhöhter Temperatur.

Die Erfindung betrifft insbesondere ein Verfahren, welches die Herstellung von amphoteren Tensiden ermöglicht, deren Gehalt an organisch gebundenem Chlor und insbesondere von Natriummono- und -dichloracetat stark vermindert ist.

Amphotere Tenside werden in großem Umfang zur Herstellung von Körperreinigungsmitteln, insbesondere zur Herstellung von Haarwaschmitteln verwendet. Man ist deshalb bestrebt, die amphoteren Tenside frei von Verunreinigungen herzustellen, welche Hautreizungen verursachen können oder in sonstiger Weise aus physiologischen Gründen unerwünscht sind. Hierzu zählen auch Restmengen an Verbindungen mit organisch gebundenem Chlor, insbesondere Natriumdichloracetat, welches mit dem Rohstoff Chloressigsäure oder deren Salzen mittelbar in das Endprodukt eingebracht wird. Versuche, den Gehalt an Natriumdichloracetat durch Anwendung verlängerter Reaktionszeiten oder höherer pH-Werte zu reduzieren, führten nicht zu einer wesentlichen Verminderung des Dichloracetatgehaltes. Die Anwendung erhöhter pH-Werte, insbesondere solcher oberhalb pH 10,5, bringt das Risiko einer zunehmenden Zersetzung der Produkte mit sich.

Die DE-OS 39 39 264 betrifft ein Verfahren zur Erniedrigung des Restgehaltes an freiem Alkylierungsmittel in wäßrigen Lösungen amphoterer oder zwitterionischer Tenside mit dem Kennzeichen, daß man die Lösungen mit Ammoniak, einer Aminosäure mit 2 bis 8 Kohlenstoffatomen oder einem Oligopeptid nachbehandelt. Durch diese Nachbehandlung soll der Restgehalt an freiem Alkylierungsmittel, insbesondere an Chloressigsäure, auf Werte unterhalb von 0,01 Gew.-% (bezogen auf Feststoffgehalt) gesenkt werden. Ein wesentlicher Nachteil dieses Verfahrens besteht jedoch darin, daß ein zusätzlicher Verfahrensschritt benötigt wird. Ein weiterer Nachteil ist darin zu erkennen, daß die Umsetzungsprodukte des Alkylierungsmittels mit Ammoniak, einer Aminosäure oder einem Oligopeptid im Verfahrensprodukt als Verunreinigung bleiben.

Die vorliegende Erfindung befaßt sich mit dem technischen Problem der Herstellung eines amphoteren Tensids, welches sich durch einen stark verminderten Gehalt an Verunreinigungen, insbesondere von Verbindungen mit organisch gebundenem Chlor, wie Natriummono- und -dichloracetat, auszeichnet. Dabei soll nach Möglichkeit ein separater zusätzlicher Verfahrensschritt zur Reinigung der Produkte vermieden werden.

Das erfindungsgemäße Verfahren ist dadurch gekennzeichnet, daß man Verbindungen der allgemeinen Formel 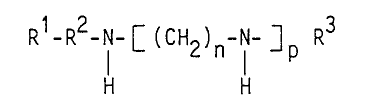
wobei
- R¹: ein gesättigter oder ungesättigter Alkylrest mit 7 bis 19 Kohlenstoffatomen ist,
- R²: ein -(CH₂)_{q}-, -O(CH₂)_{q}- oder 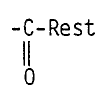 ist (q = 1 bis 3),
- R³: ein Wasserstoff- oder -CH₂CH₂-OH-Rest ist,
- n: einen Zahlenwert von 2 oder 3 und
- p: einen Zahlenwert von 1 bis 4 hat,
mit, in bezug auf die vorbezeichneten Amine, mindestens äquimolaren Mengen Chloressigsäure oder deren Salzen innerhalb eines Temperaturbereiches von 115 bis 180°C solange umsetzt, bis der Gehalt an Chloressigsäuren < 10 ppm beträgt.

In einer bevorzugten Verfahrensvariante wird in einer ersten Stufe das Amin bei 80 bis 100°C partiell oder vollständig carboxymethyliert.

In der Formel 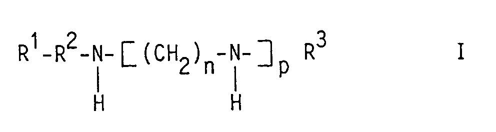
für die Ausgangsverbindung ist R¹ ein gesättigter oder ungesättigter Alkylrest mit 7 bis 19 Kohlenstoffatomen. Vorzugsweise ist R¹ ein gesättigter oder ungesättigter Alkylrest mit 9 bis 17 Kohlenstoffatomen.

R² ist ein -(CH₂)_{q}-, -O(CH₂)_{q}- oder 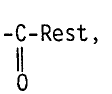
wobei
der Index q einen Zahlenwert von 1 bis 3 hat. Vorzugsweise ist der Rest R² ein -(CH₂)₂- oder ein -O(CH₂)₃-Rest.

R³ ist ein Wasserstoff- oder -CH₂CH₂-OH-Rest, wobei in diesem Fall R³ vorzugsweise die Bedeutung eines Wasserstoffrestes hat.

n hat einen Zahlenwert von 2 oder 3, vorzugsweise von 2. p hat einen Zahlenwert von 2 bis 4, vorzugsweise von 1 bis 2.

Vorzugsweise führt man die Reaktion bei einer Temperatur von 120 bis 160°C, insbesondere bei einer Temperatur von 120 bis 140°C durch. Die Reaktionsdauer, nach der der Gehalt an Mono- und Dichloressigsäure < 10 ppm ist, beträgt in Abhängigkeit von der Temperatur etwa 1 bis 10 Stunden.

Es kann von Vorteil sein zunächst in einer Vorreaktion bei 80 bis 100°C das Amin partiell oder vollständig zu carboxymethylieren und den Abbau des organisch gebundenen Chlors erst im Anschluß hieran bei der erhöhten Temperatur von mindestens 115°C vorzunehmen. Da bei der erhöhten Temperatur die Carboxymethylierungsreaktion und die Abbaureaktion des organisch gebundenen Chlors konkurrierend verlaufen, muß bei der einstufigen Reaktionsweise eine überstöchiometrische Menge Halogenalkylcarbonsäure eingesetzt werden. Führt man jedoch die Carboxymethylierungsreaktion bereits in einer ersten Stufe bei einer Temperatur von 80 bis 100°C partiell oder vollständig durch, ist es möglich, die Umsetzung mit stöchiometrischen Mengen oder mit einem nur geringen Überschuß an Halogenalkylcarbonsäure durchzuführen.

Es ist im Hinblick auf die gewählten Temperaturen erforderlich, in einem geschlossenen System, wie z.B. in einem entsprechend dimensionierten Rührautoklaven zu arbeiten.

Die Untergrenze des Temperaturbereiches von 115°C ist durch den Eintritt des Abbaus von Natriumdichloracetat gegeben. Unterhalb dieser Temperatur verläuft der Abbau nicht oder innerhalb eines Zeitraumes, der für ein wirtschaftlich durchzuführendes Verfahren nicht annehmbar ist. Die Obergrenze des Temperaturbereiches von 180°C ist durch die beginnende Zersetzung des Verfahrensproduktes bzw. der Reaktionspartner gegeben.

Mit dem erfindungsgemäßen Verfahren gelingt den Gehalt der Betainlösung an Natriummonochloracetat und Natriumdichloracetat unter die Grenze von < 10 ppm zu senken.

Das erfindungsgemäße Verfahren hat den Vorteil, daß dem Produkt zum Abbau der organischen Chlorverbindungen keine das Produkt verunreinigenden Reagenzien zugesetzt werden müssen.

Beispiele von nach dem erfindungsgemäßen Verfahren erhältlichen Verbindungen sind Amphoglycinate der allgemeinen Formel 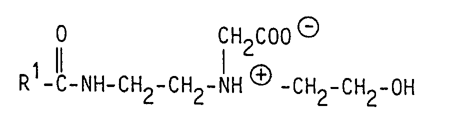
und Polybetaine 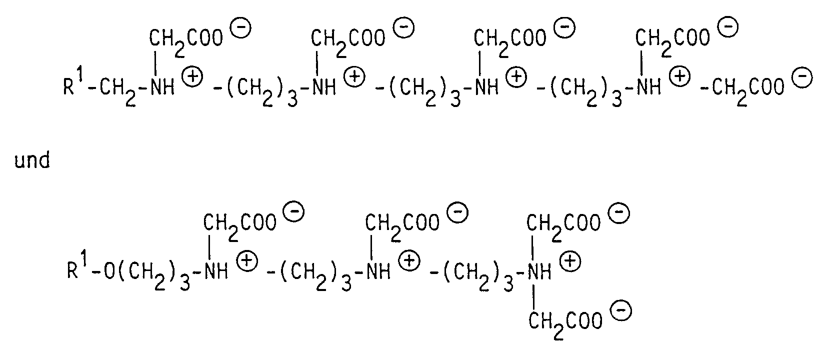
Die Reste R¹ können ein einheitliches Molgewicht oder eine Molgewichtsverteilung aufweisen. Beispiele für die Reste R¹ sind:
CH₃-(CH₂)₈-, CH₃-(CH₂)₁₀-, CH₃-(CH₂)₇-CH=CH-(CH₂)₇-, CH₃-(CH₂)₁₆-
oder, abgeleitet von der Fettsäureverteilung natürlicher Fette,
ein Gemisch von
ca. 30 Gew.-% CH₃-(CH₂)₁₄- und
ca. 70 Gew.-% CH₃-(CH₂)₁₆-
im Falle von gehärtetem Rindertalg
oder
ein Gemisch von
ca. 5 Gew.-% CH₃-(CH₂)₆-
ca. 5 Gew.-% CH₃-(CH₂)₈-
ca. 50 Gew.-% CH₃-(CH₂)₁₀-
ca. 15 Gew.-% CH₃-(CH₂)₁₂-
ca. 15 Gew.-% CH₃-(CH₂)₁₄-
ca. 10 Gew.-% CH₃-(CH₂)₁₆-
im Falle von gehärtetem Cocosöl.

Die erfindungsgemäßen Verbindungen können in vielseitiger Weise als milde Tenside in Kosmetika verwendet werden, wo sie in erster Linie als Sekundärtenside im Gemisch mit anionischen, aber auch mit weiteren milden Tensiden, wie Betainen und/oder nichtionischen Tensiden, wie Ethoxylaten von Glycerin-, Sorbitan- und anderen Zuckerestern, sowie mit Zuckertensiden, wie Alkylpolyglucosiden, eingesetzt werden. Sie können darüber hinaus auch in Textilhilfsmitteln insbesondere als Antistatika und Mittel zur Beeinflussung des Griffes von Textilien verwendet werden.

In den folgenden Beispielen wird die Herstellung von Verbindungen nach dem erfindungsgemäßen Verfahren näher erläutert.

In den Beispielen werden Amine der folgenden allgemeinen Formeln verwendet:

### Formel A:

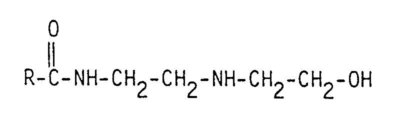

im Gemisch mit

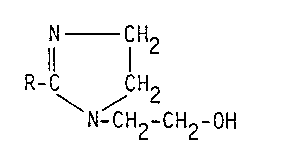

### Formel B:

R-NH-CH₂-CH₂-CH₂-NH-CH₂-CH₂-CH₂-NH₂

### Formel C:

R-NH-CH₂-CH₂-NH-CH₂-CH₂-NH-CH₂-CH₂-NH-CH₂-CH₂-NH₂

Als Natriummonochloracetat wird ein Produkt handelsüblicher Qualität eingesetzt, welches etwa 0,1 Gew.-% Natriumdichloracetat als Verunreinigung enthält. Die Gehalte an Mono- und Dichloressigsäure werden mit Hilfe einer gaschromatographischen Analyse auf folgende Weise bestimmt:
0,5 g ± 0,1 mg des Produktes werden mit 2,5 ml Ethanol 1 Minute im Ultraschallbad homogenisiert, mit 0,25 ml konzentrierter Schwefelsäure versetzt und erneut homogenisiert. Die Mischung wird für 15 Minuten auf 50°C erwärmt, nach dem Abkühlen mit 5 ml 5 gew.-%iger wäßriger Natriumchloridlösung und 5 ml Cyclohexan versetzt und 5 Minuten gerührt. Nach dem Trennen der Phasen wird aus der organischen Phase eine Probe entnommen und in einen Gaschromatographen injiziert. Die Analyse wird mit einem Hewlett Packard (HP) 5890-Gaschromatograph (Serie II) auf einer HP-20-M-Kapillarsäule in Verbindung mit einem EC-Detektor unter folgenden Bedingungen: Temperaturgradient von 75 bis 200°C = 10°C/Min., Injektor 225°C, Detektor 240°C, durchgeführt.

### Beispiel 1

### Herstellung eines Amphoglycinates aus Kokosfettsäurehydroxyethylaminoethylamid und 1 Mol Natriumchloracetat in zwei Schritten 300 g (1 Mol) eines Kokosfettsäurehydroxyethylaminoethylamids (allgemeine Formel A) aus gehärteter Kokosfettsäure mit einem durch Titration mit 0,1 normaler HCl bestimmten Stickstoffwert von 4,4 Gew.-% N werden in 428 ml Wasser dispergiert und bei 60°C mit 128,2 g (1,1 Mol) Natriumchloracetat versetzt. Das Reaktionsgemisch wird auf 95°C erwärmt. Durch die fortschreitende Reaktion sinkt der pH-Wert, er wird, sobald nötig, durch Zugabe 40 gew.-%iger NaOH auf 8,5 gehalten. Nach 7 Stunden wird eine Probe entnommen, die nach dem Abkühlen analysiert wird. Die folgenden Werte werden bestimmt:

| | |
|---|---|
| Festkörper: | 50 % |
| NaCl: | 7,5 % |
| Wasser: | 50 % |
| Natriumchloracetat: | 1,3 ppm |
| Natriumdichloracetat: | 125 ppm |

Die Hauptmenge des Ansatzes wird anschließend in einem 250-ml-Laborautoklaven für 6 Stunden auf 140°C erhitzt, wobei sich ein Druck von 2,5 bar einstellt. Nach Beendigung der Reaktion und Abkühlen des Produktes wird erneut analysiert. Die folgenden Werte werden bestimmt:

| | |
|---|---|
| Festkörper: | 50 % |
| NaCl: | 7,5 % |
| Wasser: | 50 % |
| Natriumchloracetat: | 0,1 ppm |
| Natriumdichloracetat: | 5,2 ppm |

### Beispiel 2

### Herstellung eines Amphoglycinates aus Kokosfettsäurehydroxyethylaminoethylamid und 1 Mol Natriumchloracetat in einem Schritt

Das Beispiel wird analog Beispiel 1 durchgeführt, jedoch wird nach dem Zusammenfügen der Ausgangsprodukte und der Zugabe von 10,2 ml 40 gew.-%iger NaOH der Reaktionsansatz direkt in den Laborautoklaven gegeben und für 6 Stunden auf 140°C erhitzt. Nach Beendigung der Reaktion und Abkühlen des Produktes wird analysiert. Die folgenden Werte werden bestimmt:

| | |
|---|---|
| Festkörper: | 50 % |
| NaCl: | 7,5 % |
| Wasser: | 50 % |
| Natriumchloracetat: | 0,2 ppm |
| Natriumdichloracetat: | 3,4 ppm |

### Beispiel 3

### Herstellung eines Amphocarboxyglycinates aus Kokosfettsäurehydroxyethylaminoethylamid und 2 Mol Natriumchloracetat in zwei Schritten

300 g (1 Mol) des in Beispiel 1 beschriebenen Kokosfettsäurehydroxyethylaminoethylamids werden in 556 ml Wasser dispergiert und bei 60°C mit 256,4 g (2,2 Mol) Natriumchloracetat versetzt. Das Reaktionsgemisch wird unter Rühren auf 95°C erwärmt, nach Absinken des pH-Wertes durch Zugabe 40 gew.-%iger NaOH auf einem pH-Wert von 8,5 gehalten und weitere 6 Stunden unter Rühren auf 95°C erhitzt. Dem Ansatz wird eine Probe entnommen, die nach dem Abkühlen analysiert wird. Die folgenden Werte werden bestimmt:

| | |
|---|---|
| Festkörper: | 50 % |
| NaCl: | 11,5 % |
| Wasser: | 50 % |
| Natriumchloracetat: | 0,9 ppm |
| Natriumdichloracetat: | 166 ppm |

Die Hauptmenge des Ansatzes wird anschließend in einem 250-ml-Laborautoklaven für 6 Stunden auf 140°C erhitzt, wobei sich ein Druck von 2,5 bar einstellt. Nach Beendigung der Reaktion und Abkühlen des Produktes wird erneut analysiert. Die folgenden Werte werden bestimmt:

| | |
|---|---|
| Festkörper: | 50 % |
| NaCl: | 11,5 % |
| Wasser: | 50 % |
| Natriumchloracetat: | 0,3 ppm |
| Natriumdichloracetat: | 0,4 ppm |

### Beispiel 4

### Herstellung eines Polyamphoglycinates aus Talgfettdipropylentriamin und 4 Mol Natriumchloracetat

370 g (1 Mol) Talgfettdipropylentriamin (allgemeine Formel B), dessen langkettiger Alkylrest R sich aus ca. 30 % C₁₆H₃₃ und ca. 70 % aus C₁₈H₃₇ zusammensetzt, mit einem durch Titration mit 0,1 normaler HCl bestimmten Stickstoffwert von 11,3 Gew.-% N werden in 1305 ml Wasser dispergiert und bei 70°C mit 500 g (4,3 Mol) Natriumchloracetat versetzt. Das Reaktionsgemisch wird unter Rühren auf 95°C erwärmt, nach Absinken des pH-Wertes durch Zugabe 40 gew.-%iger NaOH auf einem pH-Wert von 8,5 gehalten und weitere 6 Stunden unter Rühren auf 95°C erhitzt. Dem Ansatz wird eine Probe entnommen, die nach dem Abkühlen analysiert wird. Die folgenden Werte werden bestimmt:

| | |
|---|---|
| Festkörper: | 40 % |
| NaCl: | 11,5 % |
| Wasser: | 60 % |
| Natriumchloracetat: | 2,2 ppm |
| Natriumdichloracetat: | 234 ppm |

Die Hauptmenge des Ansatzes wird anschließend in einem 250-ml-Laborautoklaven für 2 Stunden auf 180°C erhitzt, wobei sich ein Druck von 7,5 bar einstellt. Nach Beendigung der Reaktion und Abkühlen des Produktes wird erneut analysiert. Die folgenden Werte werden bestimmt:

| | |
|---|---|
| Festkörper: | 40 % |
| NaCl: | 11,5 % |
| Wasser: | 60 % |
| Natriumchloracetat: | 0,4 ppm |
| Natriumdichloracetat: | 1,2 ppm |

### Beispiel 5

### Herstellung eines Polyamphoglycinates aus Lauryltetraethylenpentamin und 6 Mol Natriumchloracetat

358 g (1 Mol) Lauryltetraethylenpentamin (allgemeine mittlere Formel C), dessen langkettiger Alkylrest R sich zu > 90 % aus C₁₂H₂₅ zusammensetzt, mit einem durch Titration mit 0,1 normaler HCl bestimmten Stickstoffwert von 18,9 Gew.-% N werden in 1673 ml Wasser dispergiert und bei 60°C mit 757 g (6,5 Mol) Natriumchloracetat versetzt. Das Reaktionsgemisch wird unter fortgesetztem Rühren auf 95°C erwärmt, nach Absinken des pH-Wertes auf 8,5 durch Zugabe 40 gew.-%iger NaOH auf einen pH-Wert von 10 eingestellt, was nach etwa 1 Stunde der Fall ist. Das Reaktionsgemisch wird anschließend in einem Laborautoklaven für 4 Stunden auf 140°C erhitzt. Nach Beendigung der Reaktion und Abkühlen wird analysiert. Die folgenden Werte werden bestimmt:

| | |
|---|---|
| Festkörper: | 40 % |
| NaCl: | 13,5 % |
| Wasser: | 60 % |
| Natriumchloracetat: | 1,5 ppm |
| Natriumdichloracetat: | 0,9 ppm |

## Patentansprüche

1. Verfahren zur Herstellung von amphoteren Tensiden durch Umsetzung von Aminen mit Chloressigsäure oder deren Salzen in wäßriger Lösung bei erhöhter Temperatur, dadurch gekennzeichnet, daß man Verbindungen der allgemeinen Formel 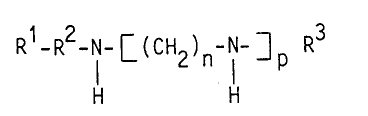 wobei
R¹ ein gesättigter oder ungesättigter Alkylrest mit 7 bis 19 Kohlenstoffatomen ist,
R² ein -(CH₂)_{q}-, -O(CH₂)_{q}- oder 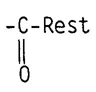 ist (q = 1 bis 3),
R³ ein Wasserstoff- oder -CH₂CH₂-OH-Rest ist,
n einen Zahlenwert von 2 oder 3 und
p einen Zahlenwert von 1 bis 4 hat,
mit, in bezug auf die vorbezeichneten Amine, mindestens äquimolaren Mengen Chloressigsäure oder deren Salzen innerhalb eines Temperaturbereiches von 115 bis 180°C solange umsetzt, bis der Gehalt an Chloressigsäuren < 10 ppm beträgt.

2. Verfahren nach Anspruch 1, dadurch gekennzeichnet, daß man in einer ersten Stufe das Amin bei 80 bis 100°C partiell oder vollständig carboxymethyliert.

3. Verfahren nach Anspruch 1, dadurch gekennzeichnet, daß man die Reaktion bei einer Temperatur von 120 bis 160°C durchführt.

4. Verfahren nach Anspruch 1, dadurch gekennzeichnet, daß man die Reaktion bei einer Temperatur von 120 bis 140°C durchführt.

## Claims

1. Process for the preparation of amphoteric surfactants by reacting amines with chloroacetic acid or salts thereof in aqueous solution at elevated temperature, characterized in that compounds of the general formula 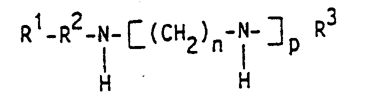 where
R¹ is a saturated or unsaturated alkyl radical with 7 to 19 carbon atoms,
R² is a -(CH₂)_{q}-, -O(CH₂)_{q}- or 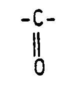 radical (q = 1 to 3),
R³ is a hydrogen or -CH₂-CH₂-OH radical,
n is a numerical value of 2 or 3 and
p is a numerical value from 1 to 4,
are reacted with amounts of chloroacetic acid, or salts thereof, which are at least equimolar with respect to the above-identified amines, within a temperature range from 115 to 180°C until the content of chloroacetic acids is < 10 ppm.

2. Process according to Claim 1, characterized in that, in a first stage, the amine is partially or completely carboxymethylated at 80 to 100°C.

3. Process according to Claim 1, characterized in that the reaction is carried out at a temperature from 120 to 160°C.

4. Process according to Claim 1, characterized in that the reaction is carried out at a temperature from 120 to 140°C.

## Revendications

1. Procédé de préparation d'agents tensio-actifs amphotères par mise en réaction d'amines avec de l'acide chloracétique ou des sels de celui-ci, en solution aqueuse, à une température élevée, caractérisé en ce qu'on fait réagir des composés répondant à la formule générale 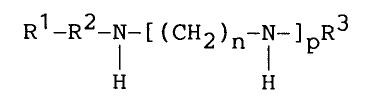 dans laquelle :
R¹ est un reste alkyle saturé ou insaturé ayant de 7 à 19 atomes de carbone,
R² est un reste -(CH₂)_{q}, -O(CH₂)_{q} ou 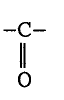 (q = 1 à 3),
R³ est un reste d'hydrogène ou le reste -CH₂CH₂-OH,
n est un nombre valant 2 ou 3, et
p est un nombre valant de 1 à 4,
avec, par rapport aux amines indiquées ci-dessus, des quantités au moins équimolaires d'acide chloracétique ou de sels de celui-ci, à une température comprise entre 115 et 180°C, jusqu'à ce que la teneur en acide chloracétique soit inférieure à 10 ppm.

2. Procédé selon la revendication 1, caractérisé en ce qu'on effectue, lors d'une première étape, la carboxyméthylation partielle ou complète de l'amine, à une température de 80 à 100°C.

3. Procédé selon la revendication 1, caractérisé en ce qu'on effectue la réaction à une température de 120 à 160°C.

4. Procédé selon la revendication 1, caractérisé en ce qu'on effectue la réaction à une température de 120 à 140°C.
